# EUROPÄISCHE PATENTSCHRIFT

(11) **EP 1 299 138 B1**
(45) Veröffentlichungstag und Bekanntmachung des Hinweises auf die Patenterteilung: **19.10.2005**
(21) Anmeldenummer: 01965081.1
(22) Anmeldetag: 06.07.2001
(51) Int. Cl.: A61M 5/44

(54) **VORRICHTUNG ZUM AUFTAUEN ODER/UND ERWÄRMEN**
DEVICE FOR DEFROSTING AND/OR HEATING UP
DISPOSITIF DE DECONGELATION ET/OU DE RECHAUFFAGE

(30) Priorität: 07.07.2000 DE 10033025
(43) Veröffentlichungstag der Anmeldung: 09.04.2003
(73) Patentinhaber: Transmed Medizintechnik GmbH & Co. KG, 33181 Bad Wünnenberg (DE)
(72) Erfinder: PELSTER, Michael, 59581 Warstein-Hirschberg (DE)
(74) Vertreter: Valentin, Ekkehard
(86) Internationale Anmeldenummer: PCT/EP2001/007798
(87) Internationale Veröffentlichungsnummer: WO 2002/004052

(56) Entgegenhaltungen:
- EP-A- 0 318 924
- WO-A-88/07384
- DE-A- 3 047 784
- US-A- 5 297 234
- US-A- 5 999 701

## Beschreibung

Die vorliegende Erfindung betrifft eine Vorrichtung zum Auftauen oder/und Erwärmen von medizinischem Gut, insbesondere von tiefgefrorenen Blutprodukten oder Injektions- bzw. Infusionslösungen zur späteren Transfusion, Retransfusion oder Injektion.

Medizinische Präparate der genannten Art werden häufig in speziellen Behältnissen und bei einer definierten Temperatur gelagert und müssen innerhalb einer bestimmten Zeit aufgetaut und/oder auf eine vorgeschriebene Endtemperatur erwärmt werden. So müssen beispielsweise aus Knochenmark oder Blut gewonnene Stammzellpräparate, die in einem Kunststoffbeutel mit einem Füllvolumen von 60-110 ml in flüssigem Stickstoff bei -196 °C gelagert werden, innerhalb von 3-6 Minuten vollständig aufgetaut werden. Die Endtemperatur eines solchen Präparates darf einen Wert von 10°C nicht überschreiten, da sich sonst die Überlebensrate der lebenserhaltenden Zellen drastisch reduziert. Auch muß sicher gestellt werden, daß das Lagerbehältnis der Stammzellen optimalen hygienischen Bedingungen ausgesetzt ist, um im Falle einer Leckage des Behältnisses eine Kontamination des Stammzellpräparates ausschließen zu können.

Üblicherweise werden die genannten medizinischen Güter, insbesondere Stammzellpräparate, mit Hilfe von offenen, auf 37-42 °C temperierten Wasserbädern aufgetaut oder erwärmt. Dabei werden die Stammzellpräparate von Hand in das Wasserbad eingetaucht und im Wasser bewegt. Durch Sichtprüfung wird dann entschieden, wann der Auftauprozeß abgeschlossen ist und der Beutel aus dem Wasser gezogen werden muß. Da es hier zu einem direkten Kontakt von Wasser und Kunststoffbeutel kommt, kann eine Kontamination des Stammzellpräparates nicht ausgeschlossen werden. Ferner kann es passieren, daß der Abschluß des Auftauens zu spät erkannt wird und die Überlebensrate der Stammzellen infolge einer erhöhten Temperatur des Gutes erheblich reduziert wird.

Zur Verbesserung dieser Situation wurde in der Vergangenheit zum Auftauen von Plasma- oder Blutkonserven in DE 37 41 051 eine Auftau- und Temperiervorrichtung vorgeschlagen. Bei der bekannten Vorrichtung wird eine üblicherweise bei -18 °C gefrorene Plasma- oder Blutkonserve zwischen zwei mit einem Temperiermedium, vorzugsweise Wasser, durchströmte Kunststoffbeutel gelegt. Die Anordnung wird über einen Exzenter in Schaukelbewegung gehalten, um eine gleichmäßige Temperaturverteilung zu gewährleisten. Die Kunststoffbeutel werden dabei auf die gewünschte Endtemperatur der Plasma -oder Blutkonserve, in der Regel 37 °C, temperiert.

Als nachteilig hat sich dabei erwiesen, daß die Temperatur des Temperiermediums bei derartigen Systemen während des Auftauens bzw. Erwärmens konstant 37 °C beträgt und zudem die Temperatur des zu temperierenden Gutes nicht ermittelt wird. Wird eine Erwärmung in einem Wasserbad oder mit Hilfe der bekannten Anordnung durchgeführt ist eine Überhitzung daher nicht auszuschließen und damit das Risiko des Unbrauchbarwerdens eines Stammzellpräparates äußerst hoch. Ein weiterer Nachteil der bekannten Systeme liegt in der Reinigung und der Sterilisation. So müssen im Fall der Vorrichtung aus DE 37 41 051 die Kunststoffbeutel und die restliche Vorrichtung keimfrei gemacht werden. Der Aufwand ist sehr hoch, da die Temperierkissen fest mit dem Temperiergerät verbunden und schlecht zugänglich sind. Aus der chemischen - durch Verwendung von Desinfektionsmitteln -, thermischen - durch hohe Differenzen von Anfangs- und Endtemperatur des zu temperierenden Gutes - und mechanischen - durch Bewegung der Kissen infolge der üblichen Handhabung des Systems - Belastung der aus Kunststoff gefertigten Temperierkissen können im Laufe der Zeit Leckagen nicht vermieden werden. Daraus resultiert wiederum eine erhebliche Beeinträchtigung der Hygiene im Umfeld der Stammzellpräparate. Sollte während des Auftauens eines Stammzellpräparates in der Vorrichtung aus DE 37 41 051 das Stammzellenpräparat infolge einer Undichtigkeit des Lagerbehältnisses auslaufen, so sind die ausgelaufenen Zellen verloren, da kein geeignetes Auffangbehältnis vorgesehen ist.

Weiterhin ist aus der DE 30 47 784 eine Vorrichtung bekannt, welche alle Merkmale des Oberbegriffs des Anspruchs 1 besitzt.

Aufgabe der vorliegenden Erfindung ist es eine Vorrichtung der eingangs genannten Art zu schaffen, die die geschilderten Nachteile nicht aufweist.

Erfindungsgemäß wird diese Aufgabe durch eine Vorrichtung nach Anspruch 1 zum Auftauen und/oder Erwärmen von in einem Behältnis befindlichen medizinischen Gut gelöst. Die Vorrichtung weist dabei mindestens ein Heizmodul und eine entnehmbare Adaptionskompresse auf, zwischen die das Behältnis gebettet und aufgetaut und/oder erwärmt wird, wobei die Vorrichtung eine Schwenkeinrichtung aufweist, die das Heizmodul während des Auftauens und/oder Erwärmens zumindest zeitweise bewegt, und eine Temperaturerfassungseinheit vorgesehen ist, die die Temperatur des medizinischen Guts erfaßt. Entsprechend der zu erreichenden Endtemperatur des medizinischen Guts ist die Adaptionskompresse vorgewärmt. Während des Temperiervorgangs gibt sie ihre Wärme an die Umgebung ab, so daß gegen Ende des Temperiervorgangs ein Gleichgewichtsszustand erreicht wird. Durch das Heizmodul wird das Behältnis mit dem medizinischen Gut erwärmt, wobei durch die Schwenkeinrichtung ein gleichmäßiges Erwärmen gewährleistet ist.

Nach einer vorteilhaften Ausführungsform weist das Heizmodul eine Heizplatte und ein Heizelement zur Erwärmung der Heizplatte auf. Die Heizplatte ist vorzugsweise aus einem Material mit guten Wärmeleiteigenschaften, wie zum Beispiel Aluminium. Dadurch wird eine besonders homogene Erwärmung des Behältnisses erreicht. Bei dem Heizelement kann es sich um einen Infrarotstrahler, eine stromdurchflossene Metallschleite oder dergleichen handeln. Das Heizelement kann dabei eine Temperatursensorik aufweisen. Hierdurch kann eine gezielte Regelung der Temperatur des Heizelementes ermöglicht werden.

Nach einer besonders vorteilhaften Ausführungsform der Erfindung ist vorgesehen, daß das Heizmodul eine mit der Heizplatte im thermischen Kontakt stehende, entnehmbare Auffangschale zur Aufnahme des Behältnisses und der Adaptionskompresse aufweist. Durch das Vorsehen einer solchen Auffangschale kann garantiert werden, daß im Falle eines Lecks im Behältnis das äufzutauende medizinische Gut nicht verloren geht, sondern gegebenenfalls noch für eine entsprechende Anwendung zu Verfügung steht. Vorzugsweise ist die Auffangschale wie die Heizplatte aus einem besonders gut die Wärme leitenden Material, zum Beispiel Aluminium, gefertigt. Die Auffangschale ist entnehmbar, so daß sie zum einen leicht ausgetauscht werden kann und zum anderen der Inhalt nicht mit der gesamten Auftauapparatur transportiert werden muß. Damit ein guter thermischer Kontakt gewährleistet ist, können an der erfindungsgemäßen Vorrichtung ein oder mehrere Fixierelemente zur Befestigung der Auffangschale an der Vorrichtung vorgesehen sein.

Die Temperaturerfassungseinheit ist nach einer vorteilhaften Variante der Erfindung beweglich, so daß sie optimal an dem Behältnis positioniert werden kann um die Temperaturmessung so genau wie möglich zu machen. Dazu weist die Adaptionskompresse wenigstens eine Aussparung auf, durch die die Temperaturerfassungseinheit - zur Erfassung der Temperatur des medizinischen Guts im Behältnis - geführt wird. Bei der Temperaturerfassungseinheit kann es sich um eine Thermospannungsmeßgerät (Thermoelement), ein Infrarotsensor (Bolometer) oder dergleichen handeln. Die Infrarotsensormessung hat dabei den Vorteil, daß die von dem Behältnis abgesonderte Strahlung ohne zeitliche Verzögerung erfaßt werden kann, so daß die Temperierung besonders effizient und fehlerunanfälling funktioniert.

Die Adaptionskompresse weist keine feste oder lösbare Verbindung zur Vorrichtung auf. So kann die Kompresse leicht ausgetauscht, gereinigt und sterilisiert werden. Zudem ist es möglich, je nach gewünschter Endtemperatur des aufzutauenden oder zu erwärmenden medizinischen Guts, Kompressen unterschiedlicher Füllvolumina und damit unterschiedlicher Erwärmungs- beziehungsweise Abkühleigenschaften zu verwenden. Hierbei ist es besonders vorteilhaft, mehrere Kompressen mit jeweils unterschiedlichen festen Füllvolumina zu verwenden, so daß die Notwendigkeit entfällt, an der Adaptionskompresse Einfüllanschlüsse vorzusehen, die wiederum die Gefahr eines Keimeintrages mit sich bringen. Die Adaptionskompresse kann vor dem eigentlichen Temperiervorgang durch das Heizmodul auf die gewünschte vorbestimmte Temperatur vorgewärmt werden. Hierzu weist die Adaptionskompresse vorteilhafterweise ein flüssiges Temperiermedium aufweist. Dadurch paßt sie sich besonders gut der Form des tiefgefrorenen Behältnisses an, welches im tiefgefrorenen Zustand zumeist eine relativ unebene Oberfläche aufweist. Hierzu besteht die Adaptionskompresse zumindest teilweise aus einem hochflexiblen Kunststoff, so daß sie sich auch während des Erwärmens/Abkühlens optimal an das aufzutauende Behältnis anpaßt.

Die Adaptionskompresse ist nach einer besonderen Ausführungsform der Erfindung sterilisierbar. Zudem kann auch die Auffangschale sterilisierbar ausgebildet sein. Hierdurch wird erreicht, daß im Falle eines Leckschlagens des Behältnisses, die aufgetaute Flüssigkeit in der Auffangschale gesammelt und später ihrer Bestimmung noch zugeführt werden kann ohne daß von der Adaptionskompresse und der Auffangschale ein Einbringen von Keimen erfolgt, wodurch die Flüssigkeit verunreinigt würde.

Die erfindungsgemäße Vorrichtung weist vorteilhafterweise eine Steuereinheit, insbesondere zur Einstellung der gewünschten Temperatur und zur Regelung des Erwärmungs- oder Auftauvorgangs, auf. Die Regelung geschieht vorteilhafterweise über einen Rechner (CPU) oder dergleichen. Vorzugsweise ist an der Steuereinheit ein Bedienfeld zur anwenderseitigen Steuerung vorgesehen, durch welches zum Beispiel die gewünschte Endtemperatur, die gewünschte Auftauzeit und dergleichen vorgegeben werden können. Vorteilhafterweise ist auch eine Signaleinheit vorgesehen, die das Erreichen der gewünschten Temperatur vorzugsweise durch ein akustisches Signal anzeigt. Somit ist es nicht erforderlich, daß der Auftauvorgang die gesamte Zeit durch eine Aufsichtsperson überwacht werden muß.

Nachfolgend werden die Erfindung und vorteilhafte Ausführungsformen anhand der Figuren 1 bis 5 schematisch näher erläutert. Es zeigen dabei:
- Fig. 1:: eine erste Ausführungsform der Erfindung in seitlicher Ansicht;
- Fig. 2:: eine weitere Ausführungsform der Erfindung in seitlicher Ansicht;
- Fig. 3:: eine spezielle Ausführungsform der Adaptionskompresse in Draufsicht;
- Fig. 4:: eine weitere Ausführungsform der Erfindung in seitlicher Ansicht;
- Fig. 5:: die Ausführungsform aus Fig. 4 in Draufsicht.

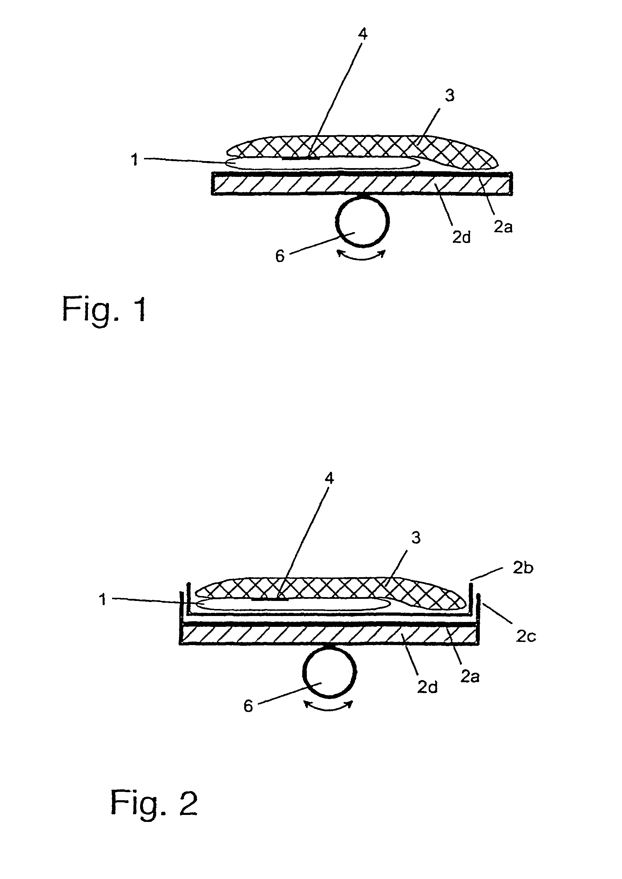

In Fig. 1 ist eine Ausführungsform der erfindungsgemäßen Auftau- beziehungsweise Aufwärmvorrichtung in seitlicher Ansicht dargestellt. Das Behältnis 1 mit dem aufzutauenden Inhalt liegt auf einem Heizmodul 2a, 2d. Das Heizmodul besteht in dieser Ausgestaltungsform aus einem Heizelement 2d und einer Heizplatte 2a. Die Heizplatte 2a wird von unten durch das Heizelement 2d erwärmt, das elektrische Energie in Wärme umsetzt. Als Heizelement 2d können unter anderem ein Infrarotstrahler oder eine stromdurchflossene Heizspirale vorgesehen sein. In dem Heizelement ist vorzugsweise eine Temperatursensorik integriert, die eine gezielte Temperaturregelung des Heizelementes ermöglicht. Die Heizplatte 2a besteht aus einem Material mit möglichst guten Wärmeleiteigenschaften, zum Beispiel Aluminium oder dergleichen, um die Wärme des Heizelements 2d möglichst gleichmäßig an das Behältnis 1 und damit den aufzutauenden Inhalt weiterzugeben. Zur Erfassung der Temperatur das zu temperierenden Gutes ist ein Temperatursensor 4 am Behältnis vorgesehen. Die Temperatur kann von einer Steuer und- Ausgabeeinheit (nicht gezeigt) ausgelesen und zur Bestimmung der Sollwerte der Temperatur des Heizmoduls verwendet werden. Das zu erwärmende Behältnis ist an der Oberseite mit einer Adaptionskompresse 3 bedeckt. Die Kompresse besteht vorzugsweise aus einem hoch flexiblen Kunststoffbeutel, der mit einem flüssigen Temperiermedium gefüllt ist. Die Wahl des Temperiermediums kann je nach einzustellender Endtemperatur erfolgen. Das Temperiermedium ist so zu wählen, daß es seine Wärme innerhalb der gewünschten Aufwärmzeit (etwa 3-4 Minuten bei Stammzellpräparaten) an die Umgebung, insbesondere das Behältnis 1 abgibt und sich ein Gleichgewicht beziehungsweise eine definierte Endtemperatur einsteltt. Weist die Adaptionskompresse infolge der chemischen, thermischen und mechanischen Belastung Verschleißerscheinungen auf, so kann diese ohne weiteres gegen eine neue Kompresse ausgetauscht werden. Die gesamte Anordnung wird mittels einer Schwenkeinrichtung 6 während des Temperiervorgangs in Bewegung gehalten. Die Schwenkeinrichtung ist mechanisch mit dem Heizmodul 2a, 2d verbunden. Mit Hilfe eines elektrischen Motors bewirkt die Schwenkeinrichtung eine periodische Schwenkbewegung der gesamten Temperieranordnung um ca. ±10°.

Mittels der Steuer- und Ausgabeeinheit wird der gesamte Temperiervorgang überwacht: Die Steuereinheit besteht aus einem Bedienfeld, einer akustischen Signaleinrichtung und einer CPU. Auf einem Bedienfeld sind zum Beispiel elektromechanische Drucktaster und Anzeigeelemente vorgesehen. Eine Recheneinheit (CPU) ist mit allen aktiven und passiven Bauelementen, der akustischen Signaleinrichtung sowie dem Bedienfeld elektrisch verbunden und steuert das Temperiersystem. Mit Hilfe einer speziellen Software können Bedien- und Steuerungsalgorithmen festgelegt oder geändert werden, so daß das komplette Temperiersystem individuell an das zu temperierende medizinische Gut angepaßt werden kann.

In Fig. 2 ist eine spezielle Ausführungsform des in Fig. 1 dargestellten Vorrichtung gezeigt. Das Heizmodul weist zusätzlich eine Auffangschale 2b auf, in die das Behältnis 1 und die Adaptionskompresse 3 gelegt werden. Die Auffangschale 2b ist ebenso wie die Heizplatte vorzugsweise aus einem Material mit guten Wärmeleiteigenschaften, wie zum Beispiel Aluminium oder dergleichen. Die Heizplatte 2a führt die Wärme an die großflächig auf ihr liegende Auffangschale 2b ab. Damit die Auffangschale 2b auf der Heizplatte nicht rutscht, kann diese mittels Fixierlaschen 2c gehalten werden.

Auffangschale 2b und Adaptionskompresse 3 sind vorzugsweise sterilisierbar ausgebildet. Dies hat einen nicht zu vemachlässigenden Vorteil. Es lassen sich so nämlich optimale hygienische Bedingungen im Umfeld der Präparate 1 herstellen. Sollte ein einzigartiges, lebenserhaltendes Präparat in der Auffangschale durch Beschädigungen am Lagerbehältnis auslaufen, so wird das Präparat vollständig durch die Schale 2b aufgefangen und kann gegebenenfalls dennoch für eine Transfusion oder dergleichen genutzt werden.

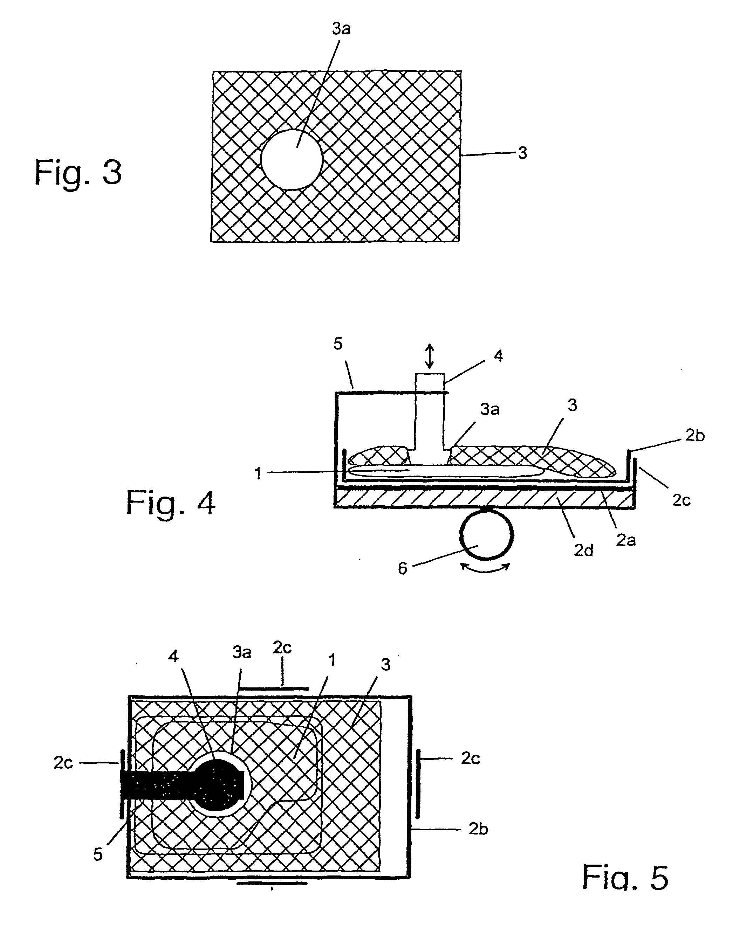

Fig. 3 bis 5 zeigen eine weitere, vorteilhafte Variante der erfindungsgemäßen Vorrichtung. Hierzu ist, wie in Fig. 3 dargestellt, in der Adaptionskompresse 3 eine Aussparung 3a vorgesehen. Durch dieses Loch 3a kann, wie in Fig. 4 dargestellt eine Temperaturmeßeinrichtung 4, 5 geführt werden. Diese setzt sich aus dem Meßkopf 4 und einem Haltebügel 5 zusammen, der fest an dem Heizmodul 2a, 2b, 2c, 2d montiert ist. Der Meßkopf 4 ist in dem Haltebügel 5 beweglich angebracht und läßt sich vertikal durch den Anwender bewegen. Hierdurch ist ein besonders leichtes Anbringen des Temperatursensors 4 am zu temperierenden Behältnis möglich. Zudem kann mit dieser Maßnahme ein Verrutschen des Temperatursensors 4 ausgeschlossen und so eine ungenaue, unter Umständen dann folgenschwere Temperaturbestimmung verhindern. Als Temperatursensor 4 ist vorzugsweise ein Infrarotsensor vorgesehen. Dies erlaubt eine besonders schnelle und dynamische Temperaturerfassung, so daß das Behältnis 1 sehr exakt temperiert und die gewünschte Endtemperatur sehr genau eingeregelt werden kann.

Das Aufwärmen/Auftauen geschieht erfindungsgemäß so, daß in einem ersten Schritt die Adaptionskompresse 3 in die Auffangschale 2b und die Auffangschale 2b wiederum auf die Heizplatte 2a gelegt wird, um Adaptionskompresse und Auffangschale mit Hilfe des Heizelementes 2d auf eine definierte Anfangstemperatur vorzuwärmen. Der Beginn der Vorwärmphase wird zum Beispiel durch Betätigung eines speziellen Drucktasters an der Steuereinheit (nicht gezeigt) aktiviert.

Ist der Vorwärmprozeß abgeschlossen, wird die Auffangschale 2b mit Adaptionskompresse 3 von der Heizplatte 2a genommen und das im Behältnis 1 befindliche, zu erwärmende Präparat zwischen Auffangschale 2b und Adaptionskompresse 3 gebettet. Anschließend wird die bestückte Auffangschale 2b auf die Heizplatte 2a gelegt und die Temperaturmeßeinrichtung 4 auf das Behältnis 1 des Präparates gesetzt. Auf diese Weise läßt sich die Temperatur des zu erwärmenden Gutes genau bestimmen.

Anschließend wird, zum Beispiel durch Betätigen eines weiteren Drucktasters an der Steuereinheit, der Erwärmprozeß gestartet. Im Verlaufe des Erwärmens wird die Auffangschale 2b durch das Heizelement 2d auf vordefinierte Temperaturen eingestellt, während die Adaptionskompresse 3 kontinuierlich auskühlt. Der Temperaturverlauf des flüssigen Temperiermediums der Kompresse 3 ist abhängig von der Wärmekapazität des Temperiermediums und seiner Anfangstemperatur. Durch die Herstellung von Adaptionskompressen 3 mit verschiedenen Füllvolumina sowie die Variation der Anfangstemperatur lassen sich somit für das jeweilige Präparat optimierte Temperaturverläufe des flüssigen Temperiermediums und damit der Adaptionskompresse 3 realisieren. Wird die angestrebte Endtemperatur des Präparates 1 erreicht, so wird dies durch die Temperaturmeßeinrichtung erkannt und zum Beispiel ein Signalton erzeugt. Dann kann der Anwender zum Beispiel durch Betätigen eines weiteren Drucktasters an der Steuereinheit (nicht gezeigt) die Erwärmung abbrechen und das Präparat 1 aus dem Erwärmsystem entnehmen.

## Patentansprüche

1. Vorrichtung zum Auftauen und/oder Erwärmen von in einem Behältnis (1) befindlichem medizinischen Gut, welche Vorrichtung mindestens ein Heizmodul (2a, 2b, 2c, 2d) und eine entnehmbare Adaptionskompresse (3) aufweist, zwischen die das Behältnis (1) gebettet und aufgetaut und/oder erwärmt wird, wobei die Vorrichtung eine Schwenkeinrichtung (6) aufweist, die das Heizmodul (2a, 2b, 2c, 2d) während des Auftauens und/ oder Erwärmens zumindest zeitweise bewegt, und eine Temperaturerfassungseinheit (4) vorgesehen ist, die die Temperatur des medizinischen Guts erfaßt,
**dadurch gekennzeichnet,**
**daß** die Adaptionskompresse (3) zumindest teilweise aus einem hochflexiblen Kunststoff besteht und keine feste oder lösbare Verbindung zur Vorrichtung aufweist.

2. Vorrichtung nach Anspruch 1, **dadurch gekennzeichnet, daß** das Heizmodul (2a, 2b, 2c, 2d) eine Heizplatte (2a) und ein Heizelement (2d) zur Erwärmung der Heizplatte (2a) aufweist.

3. Vorrichtung nach Anspruch 1 oder 2, **dadurch gekennzeichnet, daß** das Heizmodul eine mit der Heizplatte (2a) im thermischen Kontakt stehende, entnehmbare Auffangschale (2b) zur Aufnahme des Behältnisses (1) und der Adaptionskompresse (3) aufweist.

4. Vorrichtung nach Anspruch 3, **dadurch gekennzeichnet, daß** Fixierelemente (2c) zur Befestigung der Auffangschale (2b) an der Vorrichtung vorgesehen sind.

5. Vorrichtung nach einem der vorherigen Ansprüche, **dadurch gekennzeichnet, daß** die Temperaturerfassungseinheit (4) beweglich ist und die Adaptionskompresse (3) wenigstens eine Aussparung (3a) aufweist, durch die die Temperaturerfassungseinheit (4) - zur Erfassung der Temperatur des medizinischen Guts im Behältnis (1) - geführt wird.

6. Vorrichtung nach einem der vorherigen Ansprüche, **dadurch gekennzeichnet, daß** die Adaptionskompresse (3) sterilisierbar ist.

7. Vorrichtung nach einem der Ansprüche 3 bis 6, **dadurch gekennzeichnet, daß** die Auffangschale (2b) sterilisierbar ist.

8. Vorrichtung nach einem der vorherigen Ansprüche, **dadurch gekennzeichnet, daß** eine Steuereinheit, insbesondere zur Einstellung der gewünschten Temperatur und zur Regelung des Erwärmungs- oder Auftauvorgangs, vorgesehen ist.

9. Vorrichtung nach Anspruch 8, **dadurch gekennzeichnet, daß** die Steuereinheit ein Bedienfeld zur anwenderseitigen Steuerung aufweist.

10. Vorrichtung nach einem der vorherigen Ansprüche, **dadurch gekennzeichnet, daß** sie eine Signaleinheit aufweist, die das Erreichen der gewünschten Temperatur anzeigt.

11. Vorrichtung nach Anspruch 10, **dadurch gekennzeichnet, daß** es sich beim Anzeigen der gewünschten Temperatur um ein akustisches Signal handelt.

12. Vorrichtung nach einem der vorherigen Ansprüche, **dadurch gekennzeichnet, daß** sie von einer CPU gesteuert wird.

13. Vorrichtung nach einem der Ansprüche 3 bis 12, **dadurch gekennzeichnet, daß** Heizplatte (2a) und Auffangschale (2b) aus Aluminium bestehen.

14. Vorrichtung nach einem der vorherigen Ansprüche, **dadurch gekennzeichnet, daß** die Adaptionskompresse ein flüssiges Temperiermedium aufweist.

15. Vorrichtung nach einem der vorherigen Ansprüche, **dadurch gekennzeichnet, daß** die Temperaturerfassungseinheit (4) einen infrarotsensor aufweist.

16. Vorrichtung nach einem der Ansprüche 2 bis 15 **dadurch gekennzeichnet, daß** das Heizelement (2d) eine Temperatursensorik zu gezielten Regelung des Heizelementes (2d) aufweist.

## Claims

1. A device for building up and/or heating medical substance located in a container (1), which has at least one heating module (2a, 2b, 2c, 2d) and a removable adaptation compress (3), between which the container (1) is bedded and thawed and/or heated, wherein the device has a swiveling device (6) that at least temporarily moves the heating module (2a, 2b, 2c, 2d) during the thawing and/or heating process, and a temperature detector (4), which detects the temperature of the medical substance, **characterized in that** the adaptation compress (3) consists at least in part of a highly flexible plastic, and has no fixed or detachable connection to the device.

2. The device according to claim 1, **characterized in that** the heating module (2a, 2b, 2c, 2d) has a heating plate (2a) and a heating element (2d) for heating the heating plate (2a).

3. The device according to claim 1 or 2, **characterized in that** the heating module has a removable drip pan (2b) thermally contacting the heating plate (2a) to accommodate the container (1) and the adaptation compress (3).

4. The device according to claim 3, **characterized in that** fixing elements (2c) are provide for securing the drip pan (2b) to the device.

5. The device according to one of the preceding claims, **characterized in that** the temperature detector (4) is movable, and the adaptation compress (3) has at least one recess (3a) through which is routed the temperature detector (4) for determining the temperature of the medical substance in the container (1).

6. The device according to one of the preceding claims, **characterized in that** the adaptation compress (3) can be sterilized.

7. The device according to one of claims 3 to 6, **characterized in that** the drip pan (2b) can be sterilized.

8. The device according to one of the preceding claims, **characterized in that** a control unit, in particular for setting the desired temperature and regulating the heating and thawing process, is provided.

9. The device according to claim 8, **characterized in that** the control unit has a control panel for user control.

10. The device according to one of the preceding claims, **characterized in that** it has a signaling unit that indicates when the desired temperature has been reached.

11. The device according to claim 10, **characterized in that** an acoustic signal is what indicates the desired temperature.

12. The device according to one of the preceding claims, **characterized in that** it is controlled by a CPU.

13. The device according to one of claims 3 to 12, **characterized in that** the heating plate (2a) and drip pan (2b) consist of aluminum.

14. The device according to one of the preceding claims, **characterized in that** the adaptation compress has a liquid temperature control medium.

15. The device according to one of the preceding claims, **characterized in that** the temperature detector (4) has an infrared sensor.

16. The device according to one of claims 2 to 15, **characterized in that** the heating element (2d) has a temperature sensor for specifically regulating the heating element (2d).

## Revendications

1. Dispositif pour décongeler et/ou réchauffer des produits médicaux se trouvant dans un contenant (1), ledit dispositif comportant au moins un module de chauffage (2a, 2b, 2c, 2c) et une compresse d'adaptation (3) amovible, entre lesquels le contenant (1) est niché et décongelé et/ou réchauffé, le dispositif comportant un système de pivotement (6), qui déplace le module de chauffage (2a, 2b, 2c, 2d) au moins temporairement pendant la décongélation et/ou le réchauffement et une unité de détection de la température (4) qui détecte la température du produit médical étant prévue,
**caractérisé en ce que**
la compresse d'adaptation (3) est composée au moins partiellement d'une matière plastique extrêmement flexible et ne comporte aucune liaison fixe ou amovible avec le dispositif.

2. Dispositif selon la revendication 1,
**caractérisé en ce que** le module de chauffage (2a, 2b, 2c, 2d) comporte une plaque chauffante (2a) et un élément chauffant (2d) pour réchauffer la plaque chauffante (2a).

3. Dispositif selon la revendication 1 ou 2,
**caractérisé en ce que** le module de chauffage comporte une coupe collectrice (2b) amovible qui est en contact thermique avec la plaque chauffante (2a), pour loger le contenant (1) et la compresse d'adaptation (3).

4. Dispositif selon la revendication 3
**caractérisé en ce que** des éléments de fixation (2c) sont prévus sur le dispositif, pour la fixation de la coupe collectrice (2b).

5. Dispositif selon l'une quelconque des revendications précédentes,
**caractérisé en ce que** l'unité de détection de température (4) est mobile et **en ce que** la compresse d'adaptation (3) comporte au moins un évidement (3a), à travers lequel l'unité de détection de température (4) est guidée (pour détecter la température du produit médical dans le contenant (1)).

6. Dispositif selon l'une quelconque des revendications précédentes, **caractérisé en ce que** la compresse d'adaptation (3) est stérilisable.

7. Dispositif selon l'une quelconque des revendications 3 à 6, **caractérisé en ce que** la coupe collectrice (2b) est stérilisable.

8. Dispositif selon l'une quelconque des revendications précédentes,
**caractérisé en ce qu'**une unité de contrôle est prévue, notamment pour le réglage de la température souhaitée et pour la régulation du processus de réchauffement ou de décongélation.

9. Dispositif selon la revendication 8,
**caractérisé en ce que** l'unité de contrôle comporte un panneau de commande pour le contrôle par l'utilisateur.

10. Dispositif selon l'une quelconque des revendications précédentes,
**caractérisé en ce qu'**il comporte une unité de signalisation, qui indique l'atteinte de la température souhaitée.

11. Dispositif selon la revendication 10,
**caractérisé en ce que** l'indication de la température souhaitée est un signal sonore.

12. Dispositif selon l'une quelconque des revendications précédentes,
**caractérisé en ce qu'**il est contrôlé par une unité centrale.

13. Dispositif selon l'une quelconque des revendications 3 à 12,
**caractérisé en ce que** la plaque chauffante (2a) et la coupe collectrice (2b) sont en aluminium.

14. Dispositif selon l'une quelconque des revendications précédentes,
**caractérisé en ce que** la compresse d'adaptation comporte un agent liquide d'équilibrage de température.

15. Dispositif selon l'une quelconque des revendications précédentes,
**caractérisé en ce que** l'unité de détection de température (4) comporte un capteur à infrarouges.

16. Dispositif selon l'une quelconque des revendications 2 à 15,
**caractérisé en ce que** l'élément chauffant (2d) comporte un système de capteurs pour la régulation ciblée de l'élément chauffant (2d).
